(19) Europäisches Patentamt
European Patent Office
Office européen des brevets

(11) Publication number: **0 213 713 B1**

(12) **EUROPEAN PATENT SPECIFICATION**

(45) Date of publication of patent specification: **09.10.91**

(51) Int. Cl.$^5$: **C07D 487/04**, A61K 31/40, //(C07D487/04,209:00,209:00)

(21) Application number: **86305665.1**

(22) Date of filing: **23.07.86**

# (54) 2-Oxopyrrolidine pharmaceutical compounds, salts thereof, and the preparation thereof.

(30) Priority: **23.07.85 JP 161213/85**

(43) Date of publication of application:
**11.03.87 Bulletin 87/11**

(45) Publication of the grant of the patent:
**09.10.91 Bulletin 91/41**

(84) Designated Contracting States:
**CH DE FR GB IT LI**

(56) References cited:
**EP-A- 0 039 903**
**EP-A- 0 089 900**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Kurono, Masayasu**
**3-6-7, Sasao-nishi Toin-cho**
**Inabe-gun Mie-ken(JP)**
Inventor: **Hayashi, Motohide**
**2345-1410, Higashiyama-cho**
**Kasugai-shi Aichi-ken(JP)**
Inventor: **Suzuki, Tsunemasa**
**666, Minami-toyozaki Matsubase-cho**
**Shim-mashiki-gun Kumamoto-ken(JP)**
Inventor: **Miura, Kenji**
**2-59, Sekida-cho**
**Kasugai-shi Aichi-ken(JP)**
Inventor: **Kumagai, Yoshihiro**
**Mezon-choda 7, 2-2, Choda-cho**
**Kasugai-shi Aichi-ken(JP)**
Inventor: **Matsumoto, Yukiharu**
**2-3, Kano-nagai-cho**
**Gifu-shi Gifu-ken, 500(JP)**
Inventor: **Miyano, Seiji**
**2-6-13, Nagaoka Minami-ku**
**Fukuoha-shi Fukuoka-ken(JP)**
Inventor: **Sumoto, Kunihiro**
**2-4-65, Tsutsujigaoka**
**Ohnojo-shi Fukuoka-ken(JP)**

(74) Representative: **Diamond, Bryan Clive et al**
**Gee & Co., Chancery House, Chancery Lane**
**London WC2A 1QU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to novel 2-oxopyrrolidine compounds, salts therof, a process for the preparation of the compounds or salts, as well as a pharmaceutical agent comprising at least one such compound or salt and useful to prevent or cure cerebral dysfunction.

Hitherto, various studies have been made on γ- amino butyric acid (GABA) and its-derivatives to seek an effective component for curing cerebral dysfunction. During these studies, 2-oxo-1-pyrrolidineacetamide (Piracetam, 2-(pyrrolidin-2-on-1-yl) acetamide) has been discovered and has been expected to be useful, but its pharmaceutical effects have been found to be inadequate.

A primary object of the invention is to provide an effective agent for preventing or curing cerebral dysfunction, which can be substituted for the Piracetam.

A secondary object of the invention is to provide an agent for preventing or curing cerebral dysfunction, which has a strong pharmaceutical action and no or quite low toxicity.

According to the invention, a novel 2-oxopyrrolidine compound is represented by the general formula:

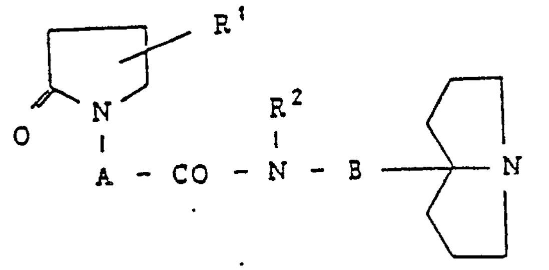

(I)

wherein $R^1$ and $R^2$ are each a hydrogen atom or a straight-chain alkyl group having 1 to 10 carbon atoms, an isopropyl, iso-butyl, s-butyl, tert-butyl or isopentyl group or a cycloalkyl group having 3 to 6 carbon atoms, A is a straight-chain alkylene group having 1 to 3 carbon atoms or a branched-chain alkylene group having 2 to 10 carbon atoms which alkylene groups may be phenyl-substituted; and B is a straight-chain alkylene group having 1 to 3 carbon atoms or a branched-chain alkylene group having 2 to 10 carbon atoms.

The invention also includes the pharmaceutically acceptable acid addition salts of these compounds.

The compounds (I) and salts thereof have an excellent cerebral metabolic activating action and cerebral function protecting action, and have a quite low toxicity.

In the compounds of Formula (I), the alkyl group may be a straight-chain alkyl radical, branched-chain alkyl radical or cycloalkyl radical. Examples for the straight-chain alkyl radicals, having 1 to 10 carbon atoms, are methyl, ethyl, n-propyl, n-butyl, n-pentyl, n-hexyl and n-decyl. Examples of the branched-chain alkyl radicals are isopropyl, isobutyl, s-butyl, t-butyl and isopentyl. Examples of the cycloalkyl radicals are cyclopropyl, cyclobutyl, cyclopentyl, and cyclohexyl. The alkylene group may be straight-chain or branched chain. Examples of straight-chain alkylene radicals, having 1 to 3 carbon atoms, are methylene, ethylene and trimethylene. Examples of branched-chain alkylene radicals, having 2 to 10 carbon atoms, are ethylidene, propylidene, butylidene, pentylidene, hexylidene, decylidene, isopropylidene, isobutylidene, isopentylidene, isobutylidene, isopentylidene, propylene, ethylethylene and ethyltrimethylene.

The salts of the compound (I) are acid addition salts having no toxicity. Suitable acids for forming a salt are hydrogen chloride, hydrogen bromide or the like hydrogen halide, sulfuric acid, phosphoric acid or the like mineral acid, acetic acid, propionic acid, gluconic acid, maleic acid, fumaric acid, tartaric acid, succinic acid, lactic acid, benzoic acid, cinnamic acid or the like organocarboxylic acid, methanesulfonic acid or the like alkylsulfonic acid, p-toluenesulfonic acid or the like arylsulfonic acid, cyclohexyl-sulfonic acid or the like cycloalkysulfonic acid, and asparaginic acid, glutamic acid, N-acetylasparaginic acid, N-acetylglutamic acid or the like amino acid.

According to the process of the invention, the compounds (I) and salts thereof can be prepared by reacting a compound represented by the formula

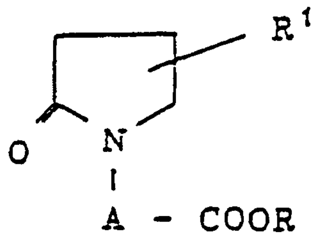

(II)

wherein $R^1$ and A have the meanings defined above, and R is an alkyl group, with a compound represented by the formula

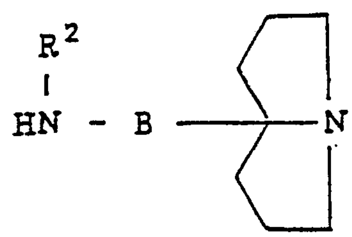

(III)

wherein $R^2$ and B are as defined above, and if necessary, converting the resulting compound into the salt.

The reaction proceeds merely by stirring both reactants in equimolar amount in the presence or absence of a solvent. As the solvent, toluene, xylene, N,N-dimethylformamide, N, N-dimethylacetamide or the like may be employed. The reaction temperature depends on the kind and other factors of the raw materials and solvent and thus it is not always constant, but 80 to 150 °C is preferable.

Each of the reactants can be prepared in a known manner. For instance, the compound of Formula (II) may be synthesized in accordance with the process disclosed in Jap. Unexamined Patent Application Gazette No. 130656/1978, and that of the Formula (III) may be synthesized in accordance with the process disclosed in Jap. Unexamined Patent Application Gazette No. 156283/1981.

The invention also includes medicaments (pharmaceutical compositions) comprising a pharmaceutically effective amount of a compound or salt according to the invention. There is no limitation on the form of the medicine and thus it may be a tablet, pill, hard capsule, soft capsule, powder, granule, suppository or the like solid medicament, or solution, suspension, emulsion or the like liquid medicament. In the case of a solid medicament a starch, lactose, glucose, calcium phophate, magnesium stearate, gum arabic or the like forming agent if necessary, smoothening or greasing agent, binding agent, breaking agent, coating agent, coloring agent or the like may be added. In the case of a liquid medicament, a stabilizer, dissolving aid, suspending agent, emulsifying agent, buffering agent, storage improvement agent of the like may be added.

The dosage amount of the compound or salt depends on various factors such as the particular compound, nature of the disease, and age and state of the patient, but for an adult, about 1 to 1000 mg/day and more particularly 5 to 30 mg/day is preferable.

The present invention has the following advantages.

By the process of the invention, the compounds and salts thereof can be prepared easily and with a relatively high yield by starting from raw materials which are known or can easily be synthesized with use of known materials.

The compounds and salts thereof according to the invention are excellent in cerebral metabolic activating action and cerebral function protecting action and show a quite low toxicity, so that they are quite useful as effective components for a cerebral dysfunction curing or preventing agent. Therefore, the agent can be orally or non-orally dosed to patients with senile dementia due to a primary cerebral degeneration, cerebral vascular dementia based on a cerebral vascular dysfunction and particularly cerebloslcerosis, or a combined type dementia based on both of these factors. The compound or salt may be dosed to a person without learning ability, amnesiac or patient with an impairment of consciousness due to injury to the head, for the purpose of appearance of activating action in the central nervous system, or preventing such diseases.

The invention will now be further explained with reference to the following Examples of preparation of

the compounds and salts, Pharmacological Test Examples thereof and Examples of making pharmaceutical preparations.

Example 1

7a-(2-oxo-1-pyrrolidineacetamidomethyl)pyrrolizidine

A mixture consisting of 14.1g (89.6 mmol) of methyl 2-oxo-1-pyrrolidineacetate and 11.4g (81.4 mmol) of 7a-(aminomethyl)pyrrolizidine was heated and stirred for 6 hours at 80 °C under argon atmosphere. After cooling, the reaction mixture was then dissolved in a suitable amount of aqueous 1.0N-hydrochloric acid and extracted 3 times with dichloromethane. The aqueous layer was rendered alkaline by the addition of sodium hydroxide solution and extracted 3 times with dichloromethane followed by drying over anhydrous magnesium sulfate. After removal of the drying agent through filtration, the solvent was evaporated under reduced pressure to obtain a yellowish oil as a free base of the desired compound. The free base was converted to the terephthalate by usual manner. Yield : 32.0g (91.1%).

Terephthalate

Melting point : 193.2 °C

| Elementary analysis : $C_{14}H_{23}N_3O_2 \cdot C_8H_6O_4$ | | | |
|---|---|---|---|
| Cal. :<br>Found : | C, 61.24;<br>C, 61.19; | H, 6.77;<br>H, 6.91; | N, 9.74<br>N, 9.58 |

IR spectrum ($\nu_{max}^{KBr}$) $cm^{-1}$:

1665, 1535, 1400

Mass spectrum (EI/DI) m/z :

166, 149, 110 (base peak)

Free base

NMR spectrum ($CDCl_3$) $\delta$ ppm :

1.07 - 3.17

(16H, m,

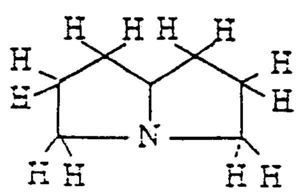

and

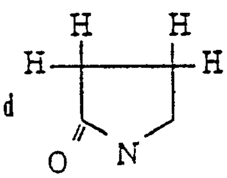

)

3.17

(2H, d, J = 5.4Hz,

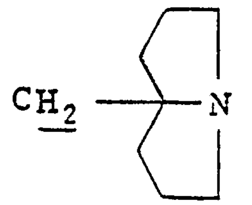 )

3.52

(2H, t, J = 7.0Hz,

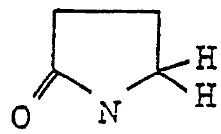 )

3.95

(2H, s,

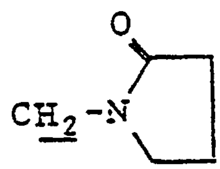 )

6.43 - 6.93 (1H, br, NH)

Examples 2 to 4

Following products were prepared by the procedure described in Example 1.

a) 7a-(2-oxo-1-pyrrolidineacetamidoethyl)pyrrolizidine Picrate

Yield : 69.3%
Melting point : 168 - 9° C

| Elementary analysis : $C_{15}H_{25}N_3O_2 \cdot C_6H_3N_3O_7$ | | | |
|---|---|---|---|
| Cal. : | C, 49.60; | H, 5.55; | N, 16.53 |
| Found : | C, 49.51; | H, 5.54; | N, 16.53 |

IR spectrum ($\nu_{max}^{KBr}$) $cm^{-1}$ :

1684, 1655, 1635, 1560, 1319
Mass spectrum (EI/DI) m/z:
279 ($M^+$), 110 (base peak)

Free base

NMR spectrum ($CDCl_3$) δ ppm :

1.45 - 3.50

(20H, m,

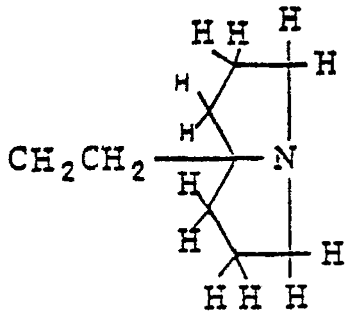

and

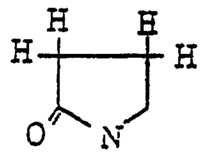

)

3.53

(2H, t, J = 7.0Hz,

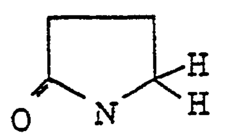

)

3.95

(2H, s,

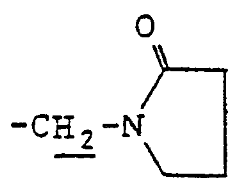

)

8.50 - 8.95 (1H, br, NH)

b) 7a-[2-(2-oxo-1-pyrrolidine)butylamidomethyl]pyrrolizidine

Telephthalate

Yield : 69.3%

Melting point : 195 - 7°C (dec.)

| Elementary analysis : $C_{16}H_{27}N_3O_2 \cdot C_8H_6O_4$ | | | |
|---|---|---|---|
| Cal. : | C, 62.73; | H, 7.24; | N, 9.14 |
| Found : | C, 62.49; | H, 7.41; | N, 9.18 |

IR spectrum ($\nu_{max}^{KBr}$) $cm^{-1}$ :

3262, 2890, 1671, 745

Mass spectrum (EI/DI) m/z:

166, 149, 110 (base peak)

Free base

NMR spectrum ($CDCl_3$) δ ppm :

0.90 (3H, t, J = 7.0Hz, $-CH_3$)

1.40 - 3.20

(18H, m,

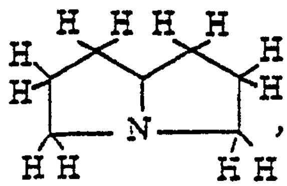
,
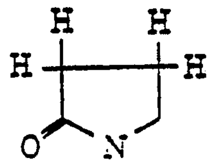
and $C\underline{H_2}CH_3$ )

3.18

(2H, d, J = 6.0Hz,

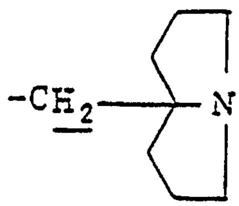
)

3.49

(2H, t, J = 7.0Hz,

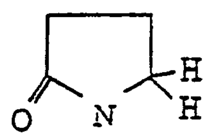
)

4.35 - 4.70

(1H, m,

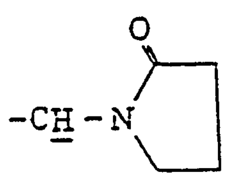
)

6.40 - 6.95 (1H, br, NH)

c) 7a-[2-(2-oxo-1-pyrrolidinyl)-2-phenylacetamidomethyl]pyrrolizidine

Telephthalate

Yield : 83.0%
Melting point : 227 - 9 ° C (dec.)

| Elementary analysis : $C_{20}H_{27}N_3O_2 \cdot 2/3\ C_8H_6O_4$ | | | |
|---|---|---|---|
| Cal. : | C, 67.28; | H, 6.91; | N, 9.29 |
| Found : | C, 62.23; | H, 7.11; | N, 9.33 |

IR spectrum ($\nu_{max}^{KBr}$) $cm^{-1}$ :

3228, 1676, 1572, 1381, 1288, 812, 746
Mass spectrum (EI/DI) m/z:
110 (base peak)

Free base

Melting point : 110 - 2 ° C
NMR spectrum ($CDCl_3$) δ ppm :
1.30 - 4.05

(18H, m,

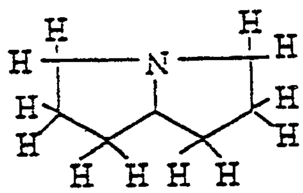

and

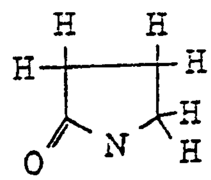

)

3.25

(2H, d, J = 6.0Hz,

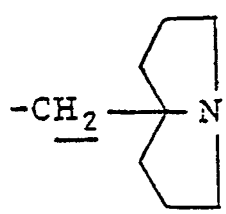

)

5.89

(1H, s, -CH-Ph)

6.20 - 6.65 (1H, br, NH)
7.43 (5H, s, aromatic protons)

Example 5

7a-(5-methyl-2-oxo-1-pyrrolidineacetamidomethyl)pyrrolizidine

This compound was obtained by use of the conditions described in Example 1 and starting from methyl 5-methyl-2-oxo-1-pyrrolidineacetate instead of methyl 2-oxo-1-pyrrolidineacetate.

NMR spectrum ($CDCl_3$) δ ppm :

1.25 (3H, d, J = 6.0Hz, $CH_3$)

1.10 - 4.00

(17H, m,

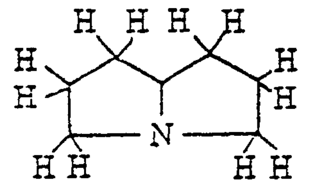

and

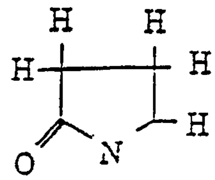

)

3.20

(2H, d, J = 6.0Hz,

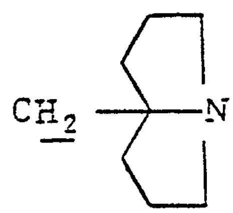

3.95

(2H, s,

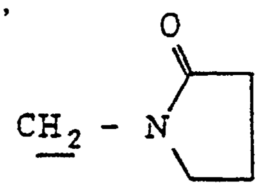

)

6.40 - 6.95 (1H, br, NH)

## Example 6

N-methyl-7a-(2-oxo-1-pyrrolidineacetamidomethyl)pyrrolizidine

A mixture consisiting of 1.60g (10.2 mmol) of methyl 2-oxo-1-pyrrolidineacetateand 1.54g (10.0 mmol) of 7a-methylaminomethylpyrrolizidine was heated and stirred for 3 hours at 140 to 150° C under argon atmosphere and then the reaction mixture was treated in the manner as described in Example 1 to obtain the desired compound.

Yield : 2.17g (77.8%)

IR spectrum ($\nu_{max}^{neat}$) $cm^{-1}$ :

2950, 2860, 1690, 1660, 1465, 1290

Mass spectrum :

(EI/DI) m/z ; 110 (base peak)

(CI/DI) m/z ; 270 [$(M+1)^+$, base peak]

NMR spectrum ($CDCl_3$) δ ppm :

1.30 - 4.90

(18H, m,

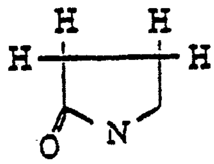

and

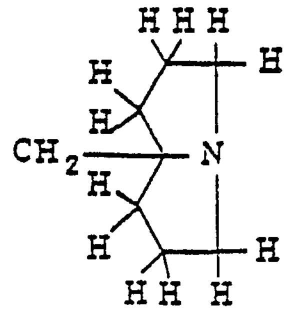

)

3.08 and 3.20 (3H, each s, N-$CH_3$)

3.55

(2H, t, J = 7.0Hz,

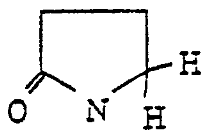

)

4.13 and 4.40

(2H, each s,

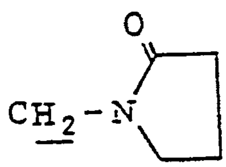

)

Pharmacological Test Example 1

(Effect on cerebral energy metabolism)

Cerebral metabolities were measured following bilateral common carotid artery occlusion in rats according to the method of Fujishima et al [ "Rinsho to Kenkyu" (translated as --Clinic and Study--), Vol. 51, page 3532, 1974, Japan.

Spontaneously hypertensive male rats (SHR), each of which had a weight of 250 to 350g and a blood pressure above 150 mm Hg, were anesthetized with ether. The common carotid artery was exposed bilaterally and double ligated simultaneously. After 1 hour, a drug to be tested was orally administered and after having lapsed 5 hours from the administration, each of the animals was exposed to microwaves (5.0 KW, 1.5 seconds). The cortex portion of brain was isolated and homogenated. Adenosin-tri-phosphate (ATP), glucose-6-phosphate (G-6-P), glucose, lactate and pyruvate concentrations in the tissue homogenate were determined by standard enzymatic methods.

As shown in the following Table 1, it is seen that the test compound (Example 1) improves cerebral energy metabolism in cerebral ischemia.

Table 1

| Test group | Dose (mg/kg) | Number of rats | A T P (μmol/g) | Lactic acid (L) (μmol/g) | Pyruvic acid (P) (μmol/g) | L/P ratio | G - 6 - P (μmol/g) | Glucose (μmol/g) |
|---|---|---|---|---|---|---|---|---|
| non-treatment | | 4 | 1.82±0.04* | 1.94±0.20*** | 390.9±18.6* | 4.93±0.40** | 169.52±11.1 | 1.44±0.05 |
| sham-operation | | 5 | 1.88±0.02** | 1.80±0.14*** | 379.8± 8.2** | 4.75±0.44** | 141.8± 4.6 | 1.54±0.11 |
| control | | 4 | 0.70±0.24 | 29.04±2.01 | 543.7± 36.1 | 54.65±26.99 | 114.8± 46.8 | 1.55±0.69 |
| product of Example 1 | 1 | 5 | 1.61±0.32 | 14.36±6.21 | 381.9±124.4 | 30.64±5.73* | 149.7±11.1 | 2.78±0.36 |
| | 10 | 5 | 1.79±0.13** | 9.01±3.24** | 429.3± 77.3 | 22.15±6.87* | 187.9±10.9 | 3.40±0.42* |
| Piracetam | 1 | 5 | 1.38±0.34 | 20.35±6.40 | 412.2± 95.0 | 44.53±6.95 | 164.7±26.7 | 2.41±0.62 |
| | 10 | 6 | 1.37±0.31 | 16.03±5.26 | 341.0± 99.7 | 62.68±29.78 | 155.0±28.3 | 2.78±0.80 |

Note 1: value is given as mean ± S.E.

2: Significantly different from the control * P<0.05, ** P<0.01, *** P<0.001

3: Piracetam: 2-(pyrrolidin-2-on-1-yl)acetoamide

Pharmaceutical Test Example 2

(Effect on gasping number after decapitation)

Cerebral protective effects of the compound according to the invention were examined in the complete ischemic model using the number of gaspings of the decapitated head of mice, in accordance with the method of Holowach-Thurston J. et al ( "Pediatr.", Vol. 8, Page 238, 1974).

Male ddY mice of 20 to 26g were used. After having lapsed 30 minutes from an oral administration of the test compound, mice were decapitated and the number of gaspings of the isolated heads were counted.

As shown in following Table 2, it is seen that the test compound has cerebral protective actions.

Table 2

| Test compounds | Dose | Number of mice | Number of gaspings |
|---|---|---|---|
| Non-treatment | | 10 | 5.0 ± 1.4 |
| Control | | 10 | 4.4 ± 1.0 |
| Product of Ex. 1 | 1 mg/kg p.o. | 10 | 5.9 ± 0.8 |
| | 10 mg/kg p.o. | 10 | 9.7 ± 1.3 (*) |
| Piracetam | 1 mg/kg p.o. | 10 | 6.3 ± 1.4 |
| | 10 mg/kg p.o. | 10 | 7.0 ± 1.2 |
| | 100 mg/kg p.o. | 10 | 6.9 ± 1.2 |

Notes :

Values are given as mean ± S.E.,

Significantly different from the control, * $p<0.01$, and

Piracetam : 2-(Pyrrolidin-2-on-1-yl)acetamide

Pharmaceutical Test Example 3

(Effect on posttraumatic consciousness disturbance)

Cerebral protection from posttraumatic consciousness distubance of mice was investigated by using the method of Manaka et al ("Igaku no Ayumi" (translated as --The Course of Medical Science--), Vol. 104, Page 253, 1978, Japan).

Male ddY mice having weight of 20 to 26g were used. After a lapse of 30 minutes from oral administration of a test compound, the mice were put into a comatose state by dropping a stick (weight : 36g) on the head from the height of 40cm. The time of appearance of the righting reflex and spontaneous movement from this comatose state were measured.

As shown in following Table 3, it is seen that the test compound has significant cerebral protective effects in consciousness disturbance.

Table 3

| Test group | Dose (mg/kg) | Number of mouse | R R T (second) | S M T (second) |
|---|---|---|---|---|
| Control | | 17 | 132.8±15.3 | 429.8±39.3 |
| Product of Example 1 | 0.1 | 10 | 120.5±13.8 | 443.2±67.1 |
| | 1 | 15 | 100.1±19.4 | 246.7±38.7(**) |
| | 10 | 12 | 102.9±24.6 | 253.4±47.7(**) |
| Piracetam | 1 | 7 | 104.4±17.4 | 387.4±129.2 |

Note

1 : Values are given as mean ± S.E.

2 : Significantly different from the control, ** $p<0.01$

3 : Piracetam

2-(Pyrrolidin-2-on-1-yl)acetamide

4 : RRT ; Righting reflex time

SMT ; Spontaneous movement time

Pharmaceutical Test Example 4

(Acute toxicity)

A test compound was given to male ddY mice having weight of 19 to 23g to observe acute toxicity and weight change. Results are shown in the following Table 4.

There was found neither a death nor any inhibition of weight increase. This means that the toxicity of

the compound according to the invention is quite low.

Table 4

| | Dose (mg/kg) | Number of mice | Weight | |
|---|---|---|---|---|
| | | | 1st day | 3rd day |
| Control | - | 10 | 21.0 ± 0.3 | 24.4 ± 0.3 |
| Product of | 100 | 10 | 20.9 ± 0.3 | 24.6 ± 0.3 |
| Ex. 1 | 1000 | 10 | 21.0 ± 0.3 | 24.4 ± 0.5 |

Pharmaceutical Agent Preparation Example 1

(Tablet)

120g of the product according to Example 1 were mixed with 1000g of lactose and 48g of hydroxypropylcellulose. The mixture was granulated with use of aqueous solution of ethanol (ethanol : water = 50 : 50), dried and screened. The resulting granules were mixed with 34g of corn starch and punched in a conventional manner to obtain 6000 tablets, each containing 20mg of the compound.

If necessary, the tablets can be coated with a film or sugar, in a conventional manner.

Pharmacological Agent Preparation Example 2

(Capsules)

| Component | Amount |
|---|---|
| Product of Example 1 | 200 (g) |
| Lactose | 1470 |
| Magnesium stearate | 30 |

The components were mixed. The resulting powder mixture was charged into hard gelatin capsules, so as to contain 170mg of the mixture and to obtain 10000 capsules, each of which contains 20mg of the compound.

Pharmaceutical Agent Preparation Example 3

(Granules)

| Component | Amount |
|---|---|
| Product of Example 1 | 400 (g) |
| Hydroxypropylcellulose | 200 |
| Corn starch | 3380 |
| Lactose | 6000 |
| Magnesium stearate | 20 |

The components were mixed and treated in a conventional manner to obtain 10kg of granules which were packed separately, each containing 0.5g of the granules (each package contains 20mg of the compound).

Pharmaceutical Agent Preparation Example 4

(Injectable Solution)

A solution was prepared in a conventional manner to the following prescription.

| Component | Amount |
|---|---|
| Product of Example 1 | 100 mg |
| Polyethylene glycol | 2 ml |
| Distilled water | remainder |
| | (total) 5 ml/vial |

Pharmaceutical Agent Preparation Example 5

(Injectable Solution)

A solution was prepared in a conventional manner to the following prescription.

| Component | Amount |
|---|---|
| Product of Example 1 | 100 mg |
| Sodium acetate | 2 mg |
| Acetic acid (to control pH to 5.8) | suitable amount |
| Distilled water | remainder |
| | (total) 5 ml/vial |

## Claims

1. A 2-oxopyrrolidine compound represented by the formula

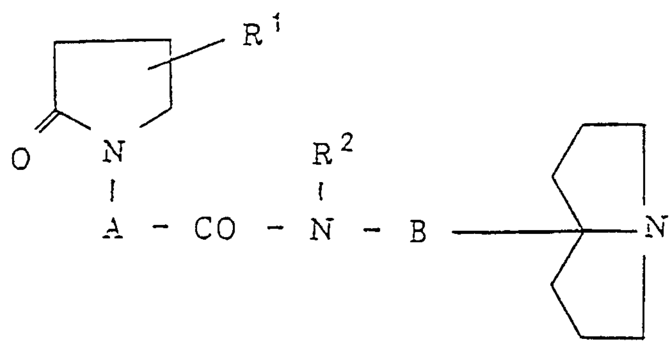 (I)

wherein $R^1$ and $R^2$ are each a hydrogen atom or a straight-chain alkyl group having 1 to 10 carbon atoms, an isopropyl, iso-butyl, s-butyl, tert-butyl or isopentyl group or a cycloalkyl group having 3 to 6 carbon atoms, A is a straight-chain alkylene group having 1 to 3 carbon atoms or a branched-chain alkylene group having 2 to 10 carbon atoms which alkylene groups may be phenyl-substituted; and B is a straight-chain alkylene group having 1 to 3 carbon atoms or branched-chain alkylene group having 2 to 10 carbon atoms; or a salt thereof.

2. A compound or salt as claimed in Claim 1, wherein said compound is 7a-(2-oxo-1-pyrrolidineacetamidomethyl)pyrrolizidine.

3. A compound or salt as claimed in Claim 1, wherein said compound is 7a-(2-oxo-1-pyrrolidineacetamidoethyl)pyrrolizidine.

4. A compound or salt as claimed in Claim 1, wherein said compound is 7a-[2-(2-oxo-1-pyrrolidine)-butylamidomethyl]pyrrolizidine.

5. A compound or salt as claimed in Claim 1, wherein said compound is 7a-[2-(2-oxo-1-pyrrolidine)-2-phenylacetamidomethyl]pyrrolizidine.

6. A compound or salt as claimed in Claim 1, wherein said compound is 7a-(5-methyl-2-oxo-1-pyrrolidineacetamidomethyl)pyrrolizidine.

7. A compound or salt as claimed in Claim 1, wherein said compound is N-methyl-7a-(2-oxo-1-pyrrolidineacetamidomethyl)pyrrolizidine.

8. A process for the preparation of a 2-oxopyrrolidine compound as claimed in Claim 1, which comprises reacting a compound represented by the formula

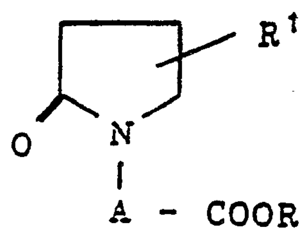

(II)

wherein $R^1$ and A are as defined in Claim 1, and R is an alkyl group, with a compound represented by the formula

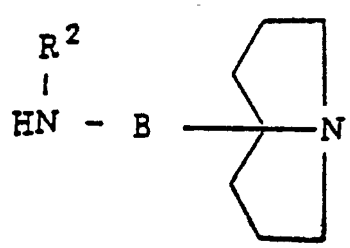

(III)

wherein $R^2$ and B are as defined in Claim 1, and if necessary, converting the resulting compound into a salt.

9. A pharmaceutical composition for preventing or curing a cerebral dysfunction, which comprises as an effective component at least one 2-oxopyrrolidine compound as claimed in any of Claims 1 to 7.

## Revendications

1. Composé 2-oxo-pyrrolidine représenté par la formule :

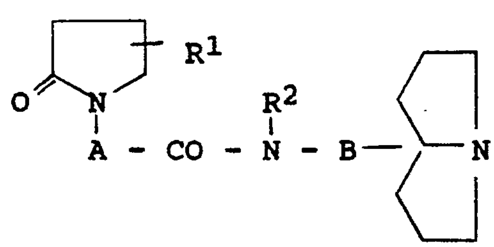

(1)

où $R^1$ et $R^2$ sont chacun un atome d'hydrogène ou un groupe alkyle à chaîne droite ayant de 1 à 10 atomes de carbone, un groupe isopropyle, iso-butyle, s-butyle tert-butyle ou isopentyle ou un groupe cycloalkyle ayant de 3 à 6 atomes de carbone, A est un groupe alkylène à chaîne droite ayant de 1 à 3 atomes de carbone ou un groupe alkylène à chaîne ramifiée ayant de 2 à 10 atomes de carbone, lesquels groupes alkylène peuvent être phényl-substitués; et B est un groupe alkylène à chaîne droite ayant de 1 à 3 atomes de carbone ou un groupe alkylène à chaîne ramifiée ayant de 2 à 10 atomes de carbone.

2. Composé ou sel selon la revendication 1, où ledit composé est la 7a-(2-oxo-1-pyrrolidineacétamidométhyl)pyrrolizidine.

3. Composé ou sel selon la revendication 1, où ledit composé est la 7a-(2-oxo-1-pyrrolidineacétamidoéthyl)pyrrolizidine.

4. Composé ou sel selon la revendication 1, où ledit composé est la 7a-[2-2-(oxo-1-pyrrolidine)-butylamidométhyl]pyrrolizidine.

5. Composé ou sel selon la revendication 1, où ledit composé est la 7a-[2-(2-oxo-1-pyrrolidine)-2-phénylacétamidométhyl]pyrrolizidine.

6. Composé ou sel selon la revendication 1, où ledit composé est la 7a-(5-méthyl-2-oxo-1-pyrrolidineacétamidométhyl)pyrrolizidine.

7. Composé ou sel selon la revendication 1, où ledit composé est la N-méthyl-7a-(2-oxo-1-pyrrolidineacétamidométhyl)pyrrolizidine.

8. Procédé de préparation d'une 2-oxopyrrolidine selon la revendication 1, comprenant de faire réagir un composé représenté par la formule

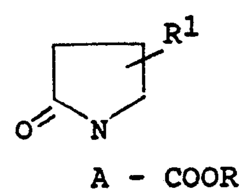

(II)

où $R^1$ et A ont la même signification que dans la revendication 1 et R est un groupe alkyle, avec un composé représenté par la formule

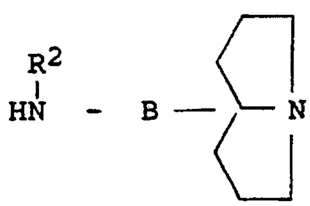

(III)

où $R^2$ et B sont comme défini dans la revendication 1, et lorsque cela est nécessaire, de convertir le composé résultant en sel.

9. Composition pharmaceutique pour empêcher ou pour guérir un dysfonctionnement cérébral, qui comprend comme agent actif au moins un composé 2-oxopyrrolidine comme revendiqué dans une quelconque des revendications 1 à 7.

**Patentansprüche**

1. Eine 2-Oxopyrrolidin-Verbindung, dargestellt durch die Formel

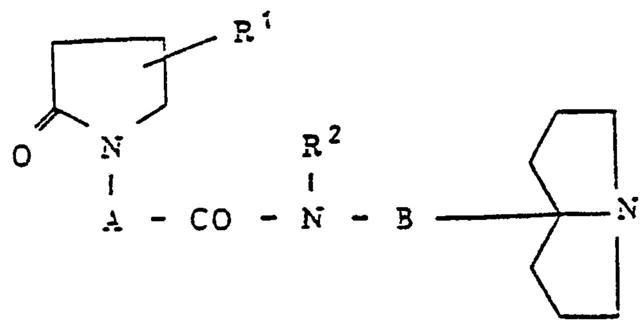

(I)

worin $R^1$ und $R^2$ je ein Wasserstoffatom oder eine geradkettige Alkylgruppe mit 1 bis 10 Kohlenstoffatomen, eine Isopropyl-, Iso-Butyl-, S-Butyl-, Tert-Butyl- oder Isopentyl-Gruppe oder eine Zykloalkyl-Gruppe mit 3 bis 6 Kohlenstoffatomen sind, A eine geradkettige Alkylen-Gruppe mit 1 bis 3 Kohlenstoffatomen oder eine verzweigte Kette einer Alkylen-Grupp mit 2 bis 10 Kohlenstoffatomen ist, wobei eine Phenyl-Substitution der Alkylen-Gruppen möglich ist; und B ist eine geradkettige Alkylen-Gruppe mit 1 bis 3 Kohlenstoffatomen oder eine verzweigte Kette einer Alkylen-Gruppe mit 2 bis 10 Kohlenstoffatomen; oder ein Salz davon.

2. Eine Verbindung oder ein Salz nach Patentanspruch 1, worin die Verbindung 7a-(2-Oxo-1-Pyrrolidinazetatamidomethyl)Pyrrolizidin ist.

3. Eine Verbindung oder ein Salz nach Patentanspruch 1, worin die Verbindung 7a-(2-Oxo-1-Pyrrolidinazetamidoäthyl)Pyrrolizidin) ist.

4. Eine Verbindung oder ein Salz nach Patentanspruch 1, worin die Verbindung 7a-[2-(2-Oxo-1-Pyrrolidin)-Butylamidomethyl]Pyrrolizidin ist.

5. Eine Verbindung oder ein Salz nach Patentanspruch 1, worin die Verbindung 7a-[2-(2-Oxo-1-Pyrrolidin)-2-Phenylazetamidomethyl]Pyrrolizidin ist.

6. Eine Verbindung oder ein Salz nach Patentanspruch 1, worin die Verbindung 7a-(5-Methyl-2-Oxo-1-Pyrrolidinazetamidomethyl)Pyrrolizidin ist.

7. Eine Verbindung oder ein Salz nach Patentanspruch 1, worin die Verbindung N-Methyl-7a-2-Oxo-1-Pyrrolidinazetamidomethyl)Pyrrolizidin ist.

8. Verfahren zur Herstellung einer 2-Oxopyrrolidin-Verbindung nach Patentanspruch 1, bestehend aus der Reaktion einer Verbindung, dargestellt durch die Formel

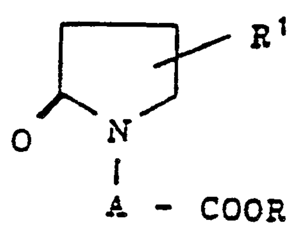

(II)

worin $R^1$ und A so, wie in Patentanspruch 1 angegeben, definiert sind, und R eine Alkyl-Gruppe ist, mit einer Verbindung, dargestellt durch die Formel

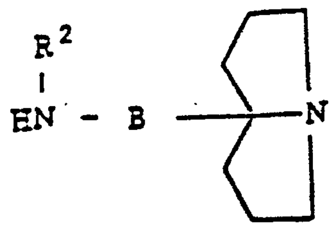

(III)

worin $R^2$ und B so, wie in Patentanspruch 1 angegeben, definiert sind, und, falls erforderlich, Umwandeln der entstandenen Verbindung in ein Salz.

9. Eine pharmazeutische Verbindung zum Verhindern ododer Heilen zerebraler Funktionsstörungen, die als Wirkkomponente mindestens eine 2-Oxopyrrolidin-Verbindung nach einem der Patentansprüche 1 bis 7 enthält.